Europäisches Patentamt

European Patent Office

Office européen des brevets

(19)

(11) Veröffentlichungsnummer: **0 276 024 B1**

(12) **EUROPÄISCHE PATENTSCHRIFT**

(45) Veröffentlichungstag der Patentschrift: **29.05.91**

(51) Int. Cl.5: **F16L 37/08, A61M 16/08**

(21) Anmeldenummer: **88101157.1**

(22) Anmeldetag: **27.01.88**

(54) **Konnektions-System für Gasleitungen mit ineinander steckbaren Verbindungselementen für Beatmungs- oder Anästhesie-Geräte.**

(30) Priorität: **29.01.87 DE 3702533**

(43) Veröffentlichungstag der Anmeldung:
**27.07.88 Patentblatt 88/30**

(45) Bekanntmachung des Hinweises auf die
Patenterteilung:
**29.05.91 Patentblatt 91/22**

(84) Benannte Vertragsstaaten:
**AT BE CH DE ES FR GB GR IT LI LU NL SE**

(56) Entgegenhaltungen:
**EP-A- 0 005 865       EP-A- 0 054 714**
**GB-A- 570 671        GB-A- 1 113 694**
**US-A- 2 389 825      US-A- 3 773 360**

(73) Patentinhaber: **Rügheimer, Erich, Prof. Dr. med.**
**Maximilianplatz 1**
**W-8520 Erlangen(DE)**

(72) Erfinder: **Rügheimer, Erich, Prof. Dr. med.**
**Maximilianplatz 1**
**W-8520 Erlangen(DE)**

(74) Vertreter: **Hafner, Dieter, Dr.rer.nat.,**
**Dipl.-Phys.**
**Ostendstrasse 132**
**W-8500 Nürnberg 30(DE)**

## Beschreibung

Die Erfindung betrifft ein Konnektions-System gemäß dem Oberbegriff des Patentanspruches 1.

Ein derartiges Konnektions-System ist aus der DE-C-30 48 223 (& EP-A-54714) bekannt.

Konnektions-Systeme dieser Art finden bei der künstlichen Beatmung von Patienten mit Hilfe einer Intubation Verwendung, beispielsweise zur Narkose mit Hilfe eines Anästhesie-Gerätes vor und während einer Operation, aber auch zur langfristigen Dauerbeatmung von Patienten, beispielsweise auf Intensivstationen. Dabei ist ein Beatmungsgerät oder ein Narkosegerät über Einatem-bzw. Ausatemleitungen in Form von Gummi- oder Kunststoffrohren an ein Y-Stück angeschlossen, wobei die Rohre bzw. Schläuche auf eine Einatemschlauchtülle bzw. eine Ausatemschlauchtülle (Beine des Y-Stückes) aufgeschoben werden. Das Y-Stück weist ein Konnektoransatzstück auf, in das ein Konnektor mit einem Tubusansatz einsteckbar ist. Am Tubusansatz des Konnektors ist dann ein in die Luftröhre eines Patienten eingeführter Endotrachealtubus aufgeschoben.

Wesentlich ist es, daß die Verbindung zwischen Y-Stück und Konnektor bei Bedarf außerordentlich rasch in einfacher Weise einhändig gelöst werden kann, während bei normalem Betrieb die Verbindung drehbeweglich bleiben soll, derart, daß der Konnektor innerhalb des Konnektoransatzstückes verdrehbar ist. Darüber hinaus kommt es ganz besonders darauf en, eine ungewollte und unerkannte Diskonnektion der Steckverbindung zwischen dem Konnektoransatzstück und dem Konnektor mit optimaler Sicherheit zu vermeiden, da sonst die Gefahr von letalen Ausgängen besteht, wenn eine derartige Diskonnektion nicht oder nicht rechtzeitig erkannt wird und der Patient infolgedessen nicht mehr richtig bzw. gar nicht mehr beatmet wird. Insbesondere zu dem Zweck, einerseits ungewollte Diskonnektionen mit Sicherheit auszuschließen und andererseits gewollte und erforderliche Diskonnektionen in einfacher Weise unter Zuhilfenahme nur einer Hand zu ermöglichen, wobei ferner durch die Sicherung der Konnektion keine Verengungen im Strömungsweg der Atemluft (oder O2) bzw. des Narkosegases (z. B. Lachgas) auftreten dürfen, ist bei dem aus der DE-C- 30 48 223 bekannten Konnektions-System weiterhin vorgesehen, mindestens ein Sicherungselement am Konnektoransatzstück quer zu dessen Längsachse anzuordnen und est als um einen Zapfen verschwenkbaren, zweiarmigen, einseitig federbelasteten Hebel auszubilden, wobei ein am Konnektoransatzstück einerseits und am äußeren Hebelarm andererseits angreifendes Federelement das Sicherungselement in seine Verriegelungsstellung vorspannt. Beispielsweise dient hierbei als Federelement eine Schraubenfeder, die in einer inneren Bohrung im äußeren Hebelarm des Sicherungselementes und in einer inneren Bohrung im Konnektoransatzstück angeordnet ist.

Infolgedessen wird das eine Verbindungselement, d.h. also das Konnektoransatzstück, mit dem zweiten Verbindungselement, d.h. dem Konnektor, lediglich durch axiales Einschieben selbsttätig verriegelt, während andererseits mit Hilfe des durch den äußeren Hebelarm gebildeten Entriegelungselements eine gewollte Diskonnektion dieser beiden Verbindungselements erreicht wird.

Bei der praktischen Anwendung dieses bekannten Konnektions-Systems im klinischen Bereich hat die Erfahrung zwar bestätigt, daß die Sicherungs- und Entriegelungselemente einwandfrei funktionieren, wobei insbesondere partielle oder totale Diskonnektionen grundsätzlich vermeidbar sind, es hat sich jedoch auf der anderen Seite herausgestellt, daß die radial über den Umfang des Konnektoransatzstückes herausragende Anordnung von Sicherungs- und Entriegelungselement zu anders gelagerten Problemen führt, welche insbesondere darin liegen, daß ein Patient vor der Intubation mit Hilfe einer Maske zu beatmen ist und beim Einführen des Endotrachealtubus in die Luftröhre des Patienten sich die erwähnte Anordnung von Sicherungs- und Entriegelungselement aufgrund deren radialen Vorspringens außerordentlich störend auswirkt. Es ist versucht worden, diese Schwierigkeiten dadurch zu umgehen, daß zusätzliche Zwischentubusstücke verwendet worden sind, die eine axiale Verlängerung des gesamten Konnektions-Systems ergaben, Derartige Maßnahmen haben sich jedoch wiederum gefährlich und störend insofern ausgewirkt, als die an das Y-Stück angeschlossenen Respirationsschläuche eine weit größere Hebelwirkung auf den Rachenraum des Patienten ausübten, und weiterhin insofern, als der Anästhesist die auf dem Gesicht des Patienten aufliegende Beatmungsmaske nicht präzise genug fixieren konnte, so daß es hierdurch zu Luftentweichungen kam.

Der vorliegenden Erfindung liegt daher die Aufgabe zugrunde, ein Konnektions-System zu schaffen, bei dem die im Vorangehenden erläuterten Probleme und Nachteile überwunden sind, d.h., bei dem das Arbeiten mit Beatmungsmasken in keiner Weise mehr behindert ist, während andererseits gleichzeitig gewährleistet bleiben soll, daß Undichtigkeiten sowie ungewollte Diskonnektionen der, ineinander gesteckten Verbindungselemente mit Sicherheit ausgeschlossen und gewollte und erforderliche Diskonnektionen in einfacher Weise einhändig durchzuführen sind.

Gleichzeitig soll vermieden und die Funktionsfähigkeit des gesamten Systems weiterverbessert werden.

Ausgehend von einem Konnektions-System gemäß dem Oberbegriff des Patentanspruchs 1 wird diese Aufgabe erfindungsgemäß durch die kennzeichnenden Merkmale dieses Anspruchs gelöst.

Vorteilhafte weitere Ausgestaltungen der Erfindung ergeben sich aus den Unteransprüchen.

Von erfindungswesentlicher Bedeutung ist die Tatsache, daß bei dem neuen Konnektions-System keinerlei störenden Elemente mehr über die Außenumfangswandung des aus Y-Stück mit Konnektoransatzstück und Konnektor mit Tubusansatz zusammengesetzten Konnektions-System hinausragen, da die Sicherungs- und Entriegelungselemente gleichebig im Niveau der Außenwandung verlaufen.

Infolgedessen können bei der praktischen Anwendung keinerlei Probleme mehr in Verbindung mit einer Beatmungsmaske und mit eventuellen Tubuszwischenstücken auftreten, wie sie weiter oben erläutert wurden.

Im übrigen wird durch die erfindungsgemäße Anordnung der Sicherungs- und Entriegelungselemente beispielsweise auch erreicht, daß sich diese in mehrfach verwendbaren Verbindungselementen befinden können, so daß die wegzuwerfenden Teile keine teuren Bauteile mehr enthalten.

Zur näheren Erläuterung der Erfindung, ihrer weiteren Merkmale und Vorteile dient die nachfolgende Beschreibung von Ausführungsbeispielen in Verbindung mit der Zeichnung.

Dabei zeigt:

Fig. 1          eine schematische Seitenansicht eines Konnektions-Systems, im wesentlichen bestehend aus Y-Stück, erstem Verbindungselement und zweiten Verbindungselement, wobei diese Komponenten in unverbundener Stellung dargestellt sind;

Fig. 1(a)          schematisch eine Draufsicht auf ein elastisches Sicherungselement, das für das erste Verbindungselement gemäß Fig.1 vorgesehen ist;

Fig. 1(b)          eine schematische Draufsicht auf einen Abschlußring für das erste Verbindungselement gemäß Fig. 1;

Fig. 2          eine weitere schematische Seitenansicht des ersten Verbindungselementes gemäß Fig. 1;

Fig. 3 und 4          jeweils weitere Ausführungsbeispiele von Steckverbindungselementen für ein Konnektions-System (in Fig. 3 in Axialschnittansicht, in Fig. 4 in schematischer Seitenansicht);

Fig. 5          eine Schnittansicht gemäß der Ebene A-A nach Fig. 3;

Fig. 6          eine Detailansicht gemäß dem Kreis in Fig. 5, mit weiteren Bauteilen; und

Fig. 7          eine weitere Ausführungsform eines Y-Stücks für eine Konnektions-System gemäß Fig. 1.

Fig. 8          eine schematische Seitenansicht eines modifizierten Konnektionssystems, wobei die einzelnen Komponenten in unverbundener Stellung dargestellt sind.

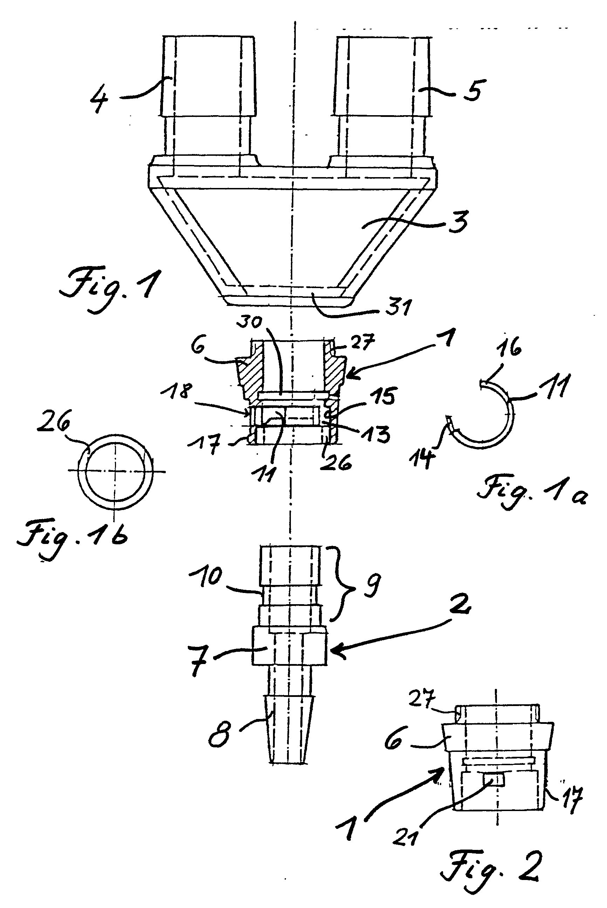

In der Fig. 1 ist schematisch in auseinandergezogener, teilweise geschnittener Ansicht ein Konnektions-System dargestellt, daß im wesentlichen aus einem Y-Stück 3, einem ersten Verbindungselement 1 sowie einem zweiten Verbindungselement 2 besteht, die ineinander steckbar ausgebildet sind.

Im speziellen Ausführungsbeispiel gemäß Fig. 1 besitzt das erste Verbindungselement 1 die Gestalt eines Konnektoransatzstückes 6 und das zweite Verbindungselement 2 die Gestalt eines Konnektors 7 mit Tubusansatz 8, der z. B. zur Verbindung mit einem (nicht gezeigten) Endotrachealtubus dient. An dem Y-Stück 3 befinden sich ferner eine Einatmungsschlauchtülle 4 sowie eine Ausatmungsschlauchtülle 5, an welche (nicht gezeigte) Atemschläuche zur Verbindung beispielsweise mit einem Beatmungs-oder einem Anästhesiegerät angeschlossen werden können.

Das Konnektoransatzstück 6 weist in seinem dem Y-Stück abgewendeten Steckverbindungsabschnitt ein Sicherungselement 11 sowie ein Entriegelungselement 12 auf, das im einzelnen aus der Fig. 6 ersichtlich ist.

Ferner ist das Konnektoransatzstück 6 an seinem dem Steckverbindungsabschnitt abgewendeten Endabschnitt mit einem äußeren Gewinde 27 versehen, mittels dessen es in eine entsprechende Öffnung 31 am gegenüberliegenden Ende des Y-Stücks einschraubbar ist.

Das Sicherungselement 11 sowie das Verriegelungselement 12 sind innerhalb des peripheren Wandungsbereichs des Konnektoransatzstückes 6 in der Weise beweglich angeordnet, daß die Sicherungs- und Entriegelungselemente weder in der Verriegelungs- noch in der Entriegelungsstellung den Außenumfang des Konnektoransatzstükkes 6 überragen.

Zu diesem Zweck ist in der Innenwandung 15 des Konnektoransatzstückes 6 eine Aussparung 13 in Form eines Ringnutsektors vorgesehen, während das Sicherungselement als ein selbstfedernder Federringsektor 11 (Fig. 1 a) ausgebildet ist.

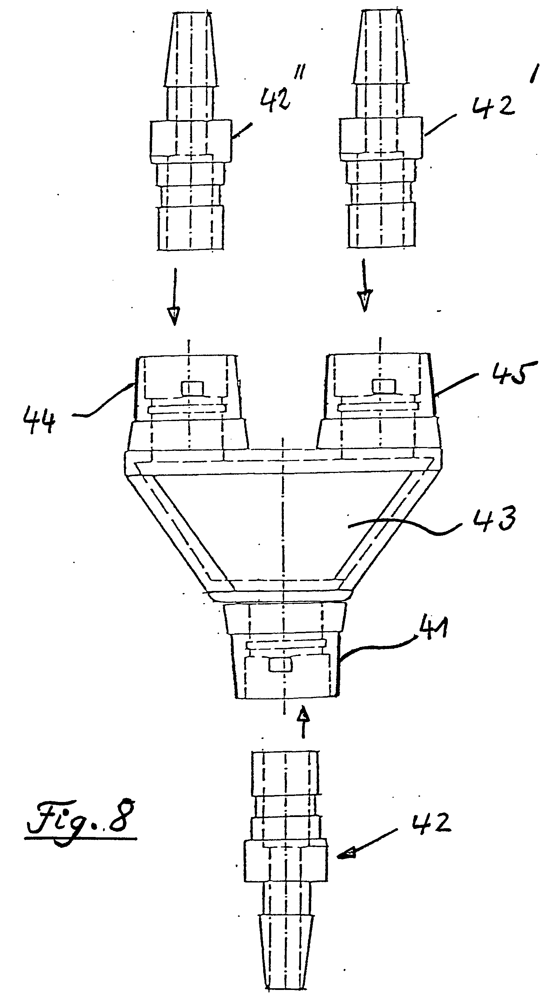

Wie dies genauer noch aus der Fig. 5 zu ersehen ist, ist der Ringnutsektor 13 im wesentlichen der Form des Federringsektors 11 entsprechend ausgebildet bzw. dieser Form angepaßt, derart, daß der Federringsektor 11 zur Erzielung einer Entriegelungsstellung zwischen Konnektoransatzstück 6 und Konnektor 7 in den Ringnutsektor 13 hineinbewegbar ist, wenn das die Federwirkung des Federringsektors 11 zeitweise überwindende Entriegelungselement 12 von Hand betätigt wird.

Im Zustand der Verriegelung dagegen, wenn Konnektoransatzstück 6 und Konnektor 7 axial ineinander geschoben sind, greift der Federringsektor 11 in die in der Außenwandung des Konnektors 7 vorgesehene, umlaufende Ringnut 10 elastisch ein, wobei sich der vordere Bereich 9 des Konnektors 7 in folge der hergestellten Steckverbindung bis zum Anschlag gegen die Außenwandung 17 des Konnektoransatzstückes 6 vollständig innerhalb dessen Innenbohrung befindet.

Im Einzelnen ist der Federringsektor 11, der sich im Bereich des Ringnutsektors 13 befindet, an seinem einen freien Ende 14 mit der Innenwandung 15 des Konnektoransatzstückes 6 fest verbunden, während der Federringsektor 11 an seinem anderen freien Ende 16 mit dem Entriegelungselement 12 befestigt ist (vgl. Fig.6).

Hierbei weist der Federringsektor 11 eine die Verriegelungsstellung definierende Vorspannung auf.

Im vorliegenden Ausführungsbeispiel entspricht der Federringssektor 11 etwa einem Kreisbogen mit dem Winkelmaß von 270°, es ist jedoch denkbar, daß auch davon abweichende Winkelmaße des Federringsektors 11 möglich sind.

Im übrigen ist die als Ringnutsektor ausgebildete Aussparung 13 in der Innenwandung 15 des Konnektoransatzstückes 6 in dessen endseitigem Steckverbindungsabschnitt nach außen hin durch einen Abschlußring 26 (vgl. auch Fig. 1(b)) begrenzt, der in eine entsprechende, endseitige Innenwandausnehmung des Konnektoransatzstückes 6 eingesetzt ist.

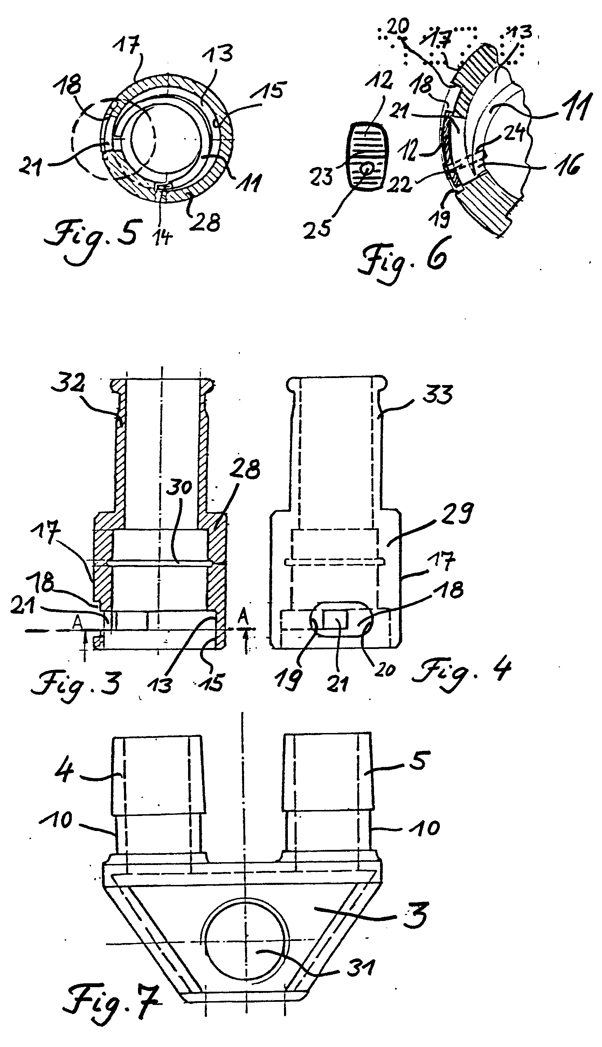

Die Außenwandung 17 des Konnektoransatzstückes 6 weist ferner eine Aussparung 18 auf (vgl. auch Fig. 4), die dem Entriegelungselement 12 zugeordnet ist. Im einzelnen ist das mit dem Sicherungselement 11 zusammenwirkende Entriegelungselement 12 innerhalb dieser Aussparung 18 über eine vorgegebene periphere Distanz zwischen zwei Endanschlägen 19 und 20 verschiebbar gelagert, wobei der erste Endanschlag 19 der Verriegelungsstellung und der zweite Endanschlag 20 der Entriegelungsstelung bezüglich der ersten und zweiten Verbindungselemente 1 und 2 entspricht.

Weiter ist nun, wie dies insbesondere aus den Fig. 3 und 5 ersichtlich, die ein Ausführungsbeispiel eines Steckverbindungselementes 28 mit einem dem Konnektoransatzstück 6 entsprechenden Steckverbindungsabschnitt darstellen, die als Ringnutsektor 13 ausgebildete Aussparung in der Innenwandung 15 des Verbindungselementes 28 über eine Öffnung 21 in der Wandung dieses Verbindungselementes mit der das Entriegelungselement 12 aufnehmenden Aussparung 18 der Außenwandung 17 dieses Verbindungselementes 28 verbunden.

Somit können Befestigungsmittel 22 vorgesehen sein (vgl. Fig. 6), die das Entriegelungselement 12 durch die Öffnung 21 in der Wandung des Verbindungselementes 28 hindurch mit dem entsprechenden freien Ende 16 des Federringsektors 11 befestigen. Wie Fig. 6 ferner zeigt, ist das Entriegelungselement 12 als ein in der ihm zugeordneten, peripheren Aussparung 18 gleitbares Gleitscheibchen ausgebildet, das von Hand betätigbar ist. Zu seiner leichteren Betätigung ist dieses Gleitscheibchen auf seiner nach außen weisenden Oberfläche mit einer Riffelung 23 versehen.

Zur Herstellung einer lösbaren Befestigung zwischen dem Entriegelungselement 12 und dem Federringsektor 11 weisen das zur Befestigung mit dem Entriegelungselement vorgesehene freie Ende 16 des Federringsektors 11 eine Gewindebohrung 24 und das Entriegelungselement 12 selbst eine entsprechend angeordnete Öffnung 25 auf, wobei die Befestigungsmittel durch eine Schraube 22 gebildet sind, mittels der das Entriegelungselement bzw. Gleitscheibchen 12 im Bereich der peripheren Öffnung 21 der Außenwandung 17 des Verbindungselementes 28 mit dem Federringsektor 11 befestigbar ist.

Durch eine handbetätigte Gleitverschiebung des Entriegelungselementes 12 von seinem ersten Endanschlag 19 zu seinem zweiten Endanschlag 20 wird bewirkt, daß die die Verriegelungsstellung definierende Vorspannung des Federringsektors 11 temporär aufgehoben wird, wobei sich dieser Federringsektor 11 vollständig in die als Ringnutsektor 13 ausgebildete Aussparung hineinbewegt. Damit ist der Zustand der Entriegelung der ineinander gesteckten Verbindungselemente erreicht, der solange besteht, bis das Entriegelungselement 12 wieder losgelassen wird. Dieses bewegt sich sodann unter der Federwirkung des Federringsektors 11 wiederum selbst in seine gegenüberliegende Endanschlagslage innerhalb der Aussparung 18, wobei sich der Federringsektor 11 von selbst in die Verriegelungsstellung bewegt, in welcher er den Querschnitt der Innenwandung 15 des Steckverbindungselementes 1 bzw. 28 verengt, wie dies aus Fig. 5 zu sehen ist, so daß der aus dem Ringnutsektor 13 in den Querschnitt hereingezogene Abschnitt des Federringsektors 11 in die Ringnut 10 in der Außenwandung des anderen Verbindungselementes 2 bzw. 4 eingreift.

Wie die Fig. 7 noch zeigt, kann das Y-Stück 3 auch so ausgebildet sein, daß das Konnektoransatzstück 6 gemäß Fig. 1 in senkrechter Richtung angesetzt werden kann, derart, daß die Achse des Konnektoransatzstückes mit der Achse des Y-Stückes einen rechten Winkel bildet. Die Gewindeöffnung 31 befindet sich daher in der Seitenwandung des Y-Stückes 3.

Im übrigen zeigt die Fig. 7 (in gleicher Weise wie die Fig. 1), daß die Einatmungsschlauchtülle 4 und die Ausatmungsschlauchtülle 5 jeweils dritte und vierte Steckverbindungselemente mit endseitigen Steckverbindungsabschnitten bilden, die dem Steckverbindungsabschnitt 9 des Konnektors 7 gemäß Fig. 1 entsprechend ausgebildet sind und eine äußere Ringnut 10 aufweisen. Infolgedessen lassen sich diese Steckverbindungselemente 4 und 5 mit den Steckverbindungselementen 28 gemäß Fig. 3 und 29 gemäß Fig. 4 in gleicher Weise verwenden, wie dies bereits anhand der Fig. 1 für die Verbindung zwischen Konnektoransatzstück und Konnektor 7 geschildert ist.

Die Steckverbindungselemente 28 und 29 weisen im übrigen noch Schlauchansatzstücke 32 und 33 auf, die zur Verbindung mit den entsprechenden Respirationsschläuchen dienen.

Die Ausbildung der Schlauchansatzstücke 32 und 33 könnte auch so sein, daß sie i. w. der Ausführung des Tubusansatzes 8 des Konnektors 7 gemäß Fig. 1 entsprechen.

Wie die Fig. 1, 3 und 4 schließlich noch zeigen, weisen die Steckverbindungselemente 6, 28 und 29 jeweils in einem auf die Sicherungs- und Entriegelungselemente 11 und 12 folgenden Innenwandungsbereich eine O-Ring-Dichtung 30 auf, was im Hinblick auf die Wiederverwendbarkeit dieser Bauteile ebenfalls recht vorteilhaft ist.

Diese O-Ring-Dichtungen 30 dienen beim Ineinanderstecken des Konnektoransatzstückes 6 mit dem Konnektor 7 bzw. der Steckverbindungselemente 28 und 29 mit den Schlauchtüllen 4 und 5 zu deren gegenseitiger Abdichtung.

Die im Vorangehenden erläuterten Bauteile des erfindungsgemäßen Konnektions-Systems können im übrigen sämtlich oder teilweise aus einem Kunststoff hergestellt sein.

Das in Fig. 8 dargestellte Konnektionssystem ist dadurch gegenüber dem in Fig. 1 dargestellten modifiziert, daß die Einatmungsschlauchtülle und Ausatmungsschlauchtülle 4, 5 durch Schlauchtüllen 44, 45 ersetzt sind, in die Schlauchtüllenkonnektoren 42', 42" eingeführt und dort verrastet werden können. Dadurch ergibt sich eine bauliche Vereinfachung gegenüber dem in Fig. 1 dargestellten System, die den Anschluß von dünnen Kinderschläuchen überhaupt erst möglich macht und die Herstellung des Systems verbilligt.

Auf eine detaillierte Beschreibung der Ausführungsform gemäß Fig. 8 kann verzichtet werden, da die Systemkomponenten 41 und 42 bzw. 44, 45, 42', 42" den Komponenten 1 und 2 in Fig. 1 völlig entsprechen.

BEZUGSZEICHENLISTE

1 erstes Verbindungselement
2 zweites Verbindungselement
3 Y-Stück
4 Einatmungsschlauchtülle
5 Ausatmungsschlauchtülle
6 Konnektoransatzstück
7 Konnektor
8 Tubusansatz
9 vorderer Bereich des Konnektors 7
10 Ringnut (im vorderen Bereich von 7)
11 Sicherungselement (Federringsektor)
12 Entriegelungselement
13 Aussparung (Ringnutsektor)
14 erstes freies Ende von 11
15 Innenwandung von 1 bzw. 6
16 zweites freies Ende von 11
17 Außenwandung von 1 bzw. 6
18 Aussparung in 17 für 12
19 erster Endanschlag von 12
20 zweiter Endanschlag von 12
21 Öffnung in 1 bzw. 6
22 Befestigungsmittel
23 Riffelung von 12
24 Gewindebohrung in 16
25 Öffnung von 12
26 Abschlußring
27 Gewinde von 6
28 fünftes Steckverbindungselement
29 sechstes Steckverbindungselement
30 O-Ring-Dichtung
31 Öffnung in 3
32 Schlauchansatzstück
33 Schlauchansatzstück

Ansprüche

1.  Beatmungs- oder Anästhesiegerät, mit Gasleitungen und einem Konnektions-System für diese Gasleitungen, wobei dieses Konnektions-System mindestens eine Steckverbindung aufweist, die aus zwei ineinander steckbaren, im wesentlichen rohrförmigen Verbindungselementen (1,2) gebildet ist, wobei ein erstes (1) der Verbindungselemente ein Sicherungselement (11), das in eine in einer Außenwandung eines zweiten (2) der Verbindungselemente umlaufende Ringnut (10) unter Federwirkung eingreift, und ein handbetätigbares Entriegelungselement (12) aufweist, das zum Herausbewegen des Sicherungselementes (11) aus der umlaufenden Ringnut (10) dient und wobei die Verbindungselemente gegeneinander abgedichtet und frei verdrehbar ausgebildet sind,

dadurch gekennzeichnet,

daß das Sicherungselement als selbstfedernder Federringsektor (11) ausgebildet ist, dem eine erste Aussparung (13) in der Innenwandung des ersten (1 bzw. 28) der Verbindungs-elemente zugeordnet ist, das Entriegelungsele-ment als ein im wesentlichen flächiges, am Federringsektor (11) befestigtes Entriegelungselement (12) ausgebildet ist, dem eine zweite Aussparung (18) in der Außenwandung des ersten (1 bzw. 28) der Verbindungselemente zugeordnet ist, und das Entriegelungselement (12) auf seiner nach außen weisenden Oberfläche mit einer Riffelung (23) versehen ist und im Bereich der zweiten Aussparung (18) in der Umfangsrichtung des einen (1 bzw. 28) der Verbindungselemente in der Weise gleitend verschiebbar ist, daß der Federringsektor (11) abwechselnd zur Entriegelung der Verbindung in die erste Aussparung (13) hineinbewegbar und zur Verriegelung der Verbindung aus dieser wieder herausbewegbar ist.

2. Gerät nach Anspruch 1,

dadurch gekennzeichnet,

daß die erste Aussparung (13) in der Innen-wandung (15) des ersten Verbindungselemen-tes (1 bzw. 28) dem Federringsektor (11) entsprechend als Ringnutsektor ausgebildet ist.

3. Gerät nach Anspruch 2,

dadurch gekennzeichnet,

daß der Federringsektor (11) an seinem einen Ende (14) mit der Innenwandung (15) des ersten Verbindungselements (1 bzw. 28) fest verbunden und an seinem anderen Ende (16) an dem Entriegelungselement (12) befestigt ist.

4. Gerät nach einem der Ansprüche 1 bis 3,

dadurch gekennzeichnet,

daß der Federringsektor (11) eine die Verriege-lungsstellung definierende Vorspannung auf-weist.

5. Gerät nach einem der vorangehenden Ansprüche,

dadurch gekennzeichnet,

daß das Entriegelungselement (12) innerhalb der zugeordneten zweiten Aussparung (18) in der Außenwandung (17) des ersten Verbin-dungselementes (1 bzw. 28) über eine vorgegebene Distanz in dessen Umfangsrichtung zwischen zwei Endanschlägen (19, 20) verschiebbar ist, wobei der erste Endanschlag (19) der Verriegelungsstellung und der zweite Endanschlag (20) der Entriegelungsstellung entsprechen.

6. Gerät nach Anspruch 5,

dadurch gekennzeichnet,

daß die als Ringnutsektor ausgebildete erste Aussparung (13) in der Innenwandung des ersten Verbindungselements (1 bzw. 28) über eine Öffnung (21) in der Wandung des ersten Verbindungselements (1 bzw. 28) mit der das Entriegelungselement (12) aufnehmenden zweiten Aussparung (18) in der Außenwandung (17) des ersten Verbindungselements (1 bzw. 28) verbunden ist.

7. Gerät nach Anspruch 6,

dadurch gekennzeichnet,

daß Befestigungsmittel (22) vorgesehen sind, die das Entriegelungselement (12) durch die Öffnung (21) in der Wandung des ersten Verbindungselements (1 bzw. 28) hindurch mit dem entsprechenden Ende (16) des Federringsektors (11) befestigen.

8. Gerät nach Anspruch 7,

dadurch gekennzeichnet,

daß die Befestigungsmittel (22) für das Entrie-gelungselement (12) lösbar angebracht sind.

9. Gerät nach Anspruch 7 oder 8,

dadurch gekennzeichnet,

daß das Entriegelungselement (12) als ein Gleitscheibchen ausgebildet ist.

10. Gerät nach einem der Ansprüche 8 bis 9,

dadurch gekennzeichnet,

daß das zur Befestigung mit dem Entriegelungselement (12) vorgesehene andere Ende (16) des Federringsektors (11) eine Gewindebohrung (24) und das Entriegelungselement (12) selbst eine entsprechend angeordnete Öffnung (25) aufweisen und daß die Befestigungsmittel (22) durch eine Schraube gebildet sind, mittels der das Entriegelungselement (12) im Bereich der Öffnung (21) der Wandung des ersten Verbindungselements (1 bzw. 28) mit dem Federringsektor (11) befestigt ist.

11. Gerät nach einem der Ansprüche 2 bis 10,

dadurch gekennzeichnet,

daß die als Ringnutsektor ausgebildete erste Aussparung (13) in der Innenwandung (15) des ersten Verbindungselements (1) in dessen dem freien Ende des Verbindungselements zugeordneten Steckverbindungsabschnitt nach außen hin durch einen Abschlußring (26) begrenzt ist, der in eine entsprechende, endseitige Innenwandausnehmung des ersten Verbindungselementes (1) einsetzbar ist.

12. Gerät nach Anspruch 11,

dadurch gekennzeichnet,

daß zur Fixierung des einen Endes (14) des Federringsektors (11) mit der Innenwandung (15) des ersten Verbindungselements (1) ein Fixierstift dient, für den achsparallele Einführbohrungen sowohl im betreffenden Federringsektorende als auch im Wandungsabschnitt des ersten Verbindungselements im Bereich des entsprechenden Ringnutsektorendes vorgesehen sind.

13. Gerät nach Anspruch 12,

dadurch gekennzeichnet,

daß der Fixierstift auch zur Fixierung des Abschlußringes (26) dient, der hierzu ebenfalls eine achsparallele Einführbohrung für den Fixierstift aufweist.

14. Gerät nach Anspruch 12 oder 13,

dadurch gekennzeichnet,

daß der Fixierstift ein Metall-, insbesondere ein Stahlstift ist.

15. Gerät nach einem der vorhergehenden Ansprüche,

dadurch gekennzeichnet,

daß der Federringsektor (11) aus kunststoffummanteltem Metall, insbesondere aus kunststoffummanteltem Stahl, besteht.

16. Gerät nach einem der Ansprüche 1 bis 14,

dadurch gekennzeichnet,

daß der Federringsektor (11) nur aus Kunststoff besteht.

17. Gerät nach einem der vorhergehenden Ansprüche,

dadurch gekennzeichnet,

daß sich der Federringsektor (11) über einen Kreisbogen mit dem Bogenmaß 3/2 $\pi$ erstreckt.

18. Gerät nach einem der vorhergehenden Ansprüche,

dadurch gekennzeichnet,

daß das erste Verbindungselement (1 bzw. 28) ein Konnektoransatzstück (6) bildet, an dessen freies Ende ein Y-Stück (3) mit einer Einatmungsschlauchtülle (4) und einer Ausatmungsschlauchtülle (5) angeschlossen ist, und das zweite Verbindungselement (2) einen Konnektor (7) mit Tubusansatz für einen Endotrachealtubus bildet.

19. Gerät nach Anspruch 18,

dadurch gekennzeichnet,

daß das Konnektoransatzstück (6) für das Y-Stück (3) an seinem einen Endabschnitt ein Gewinde (27) zum Herstellen einer Schraubverbindung mit dem Y-Stück (3) aufweist.

20. Gerät nach einem der Ansprüche 18 oder 19,

dadurch gekennzeichnet,

daß die Einatmungsschlauchtülle (4) und die Ausatmungsschlauchtülle (5) des Y-Stücks (3) jeweils dritte und vierte Steckverbindungselemente mit endseitigen Steckverbindungsabschnitten bilden, die dem Steckverbindungsabschnitt (9) des Konnektors (7) entsprechend ausgebildet sind, und daß fünfte und sechste Steckverbindungselemente (28, 29) als Schlauchansatzstücke vorgesehen sind, deren jeweilige endseitige Steckverbindungsabschnitte dem Steckverbindungsabschnitt des Konnektoransatzstückes (6) entsprechend ausgebildet sind.

21. Gerät nach einem der Ansprüche 18 - 20,

   dadurch gekennzeichnet,

   daß die ersten, fünften und sechsten Steckverbindungselemente (6, 28, 29) in einem auf die Sicherungs- und Entriegelungselemente (11, 12) folgenden Innenwandungsbereich jeweils eine O-Ring-Dichtung (30) aufweisen.

## Claims

1. An apparatus for anaesthesia or artificial respiration, With gas lines and a connection system for these gas lines, the said connection system having at least one plug-in connection consisting of two substantially tubular connecting elements (1, 2) which are adapted to be plugged one into the other, the first (1) of the connecting elements comprising a securing element (11) which engages with spring action an annular groove (10) encircling an outer wall of a second (2) of the connecting elements, and a manually operable releasing element (12) which serves to move the securing element (11) from the encircling annular groove (10), the connecting elements being sealed in respect of each other and being constructed to be freely rotatable, characterised in that the securing element is constructed as a self-springing segment of a spring ring (11) with which a first recess (13) in the inner wall of the first (1 or 28) of the connecting elements is associated, the releasing element is constructed as a substantially flat releasing element (12) which is fixed on the segment (11) of spring ring and with which a second recess (18) in the outer wall of the first (1 or 28) of the connecting elements is associated, the releasing element (12) having on its outer surface a ribbing (23) and being displaceable in a sliding fashion in the region of the second recess (18) in the peripheral direction of one (1 or 28) of the connecting elements so that the spring ring segment (11) is adapted for movement alternately into the first recess (13) to release the connection and out of the said recess again to lock the connection.

2. An apparatus according to Claim 1, characterised in that the first recess (13) is constructed as a segment of an annular groove in the inner wall (15) of the first connecting element (1 or 28) to correspond to the spring ring segment (11).

3. An apparatus according to Claim 2, characterised in that at one end (14) the spring ring segment (11) is rigidly connected to the inside wall (15) of the first connecting element (1 or 28) while at its other end (16) it is fixed to the releasing element (12).

4. An apparatus according to one of Claims 1 to 3, characterised in that the spring ring segment (11) has an initial tension which defines the locking position.

5. An apparatus according to one of the preceding Claims, characterised in that the releasing element (12) is displaceable within the associated second recess (18) in the outer wall (17) of the first connecting element (1 or 28) over a given distance in its peripheral direction and between two end stops (19, 20), the first end stop (19) corresponding to the locking position while the second end stop (20) corresponds to the releasing position.

6. An apparatus according to Claim 5, characterised in that the first recess (13) which is constructed as a segment of an annular groove in the inside wall of the first connecting element (1 or 28) is connected by an aperture (21) in the wall of the first connecting element (1 or 28) to the second recess (18) which accommodates the releasing element (12) and which is provided in the outer wall (17) of the first connecting element (1 or 28).

7. An apparatus according to Claim 6, characterised in that fixing means (22) are provided which, through the aperture (21) in the wall of the first connecting element (1 or 28), connect the releasing element (12) to the corresponding end (16) of the spring ring segment (11).

8. An apparatus according to Claim 7, characterised in that the fixing means (22) for the releasing element (12) are separably attached.

9. An apparatus according to Claim 7 or 8, characterised in that the releasing element (12) is constructed as a sliding disc.

10. An apparatus according to one of Claims 8 to 9, characterised in that the other end (16) of the spring ring segment (11) which is provided for attachment to the releasing element (12) comprises a screw-threaded bore (24) while the releasing element (12) itself comprises a correspondingly disposed aperture (25) and in that the fixing means (22) are constituted by a screw by means of which the releasing element (12) is connected to the spring ring segment (11) in the region of the aperture (21) in the wall of the first connecting element (1 or 28).

11. An apparatus according to one of Claims 2 to 10, characterised in that in its plug-in connection portion which is associated with the free end of the connecting element, the first recess (13) which is constructed as a segment of an annular groove in the inside wall (15) of the first connecting element (1) is bounded outwardly by a closure ring (26) adapted to be inserted into a corresponding recess in the end of the inside wall of the first connecting element (1).

12. An apparatus according to Claim 11, characterised in that for fixing one end (14) of the spring ring segment (11) to the inside wall (15) of the first connecting element (1) a fixing pin is used for which axially parallel insertion bores are provided both in the relevant spring ring segment end and also in the wall portion of the first connecting element in the region of the corresponding annular groove segment end.

13. An apparatus according to Claim 12, characterised in that the fixing pin also serves to secure the closure ring (26) which to this end likewise comprises an axially parallel insertion bore for the fixing pin.

14. An apparatus according to Claim 12 or 13, characterised in that the fixing pin is a metal and in particular a steel pin.

15. An apparatus according to one of the preceding Claims, characterised in that the spring ring segment (11) consists of synthetic plastics sheathed metal, particularly synthetic plastics sheathed steel.

16. An apparatus according to one of Claims 1 to 14, characterised in that the spring ring segment (11) consists solely of synthetic plastics material.

17. An apparatus according to one of the preceding Claims, characterised in that the spring ring segment (11) extends over an arc having the radians measure $3/2\pi$.

18. An apparatus according to one of the preceding Claims, characterised in that the first connecting element (1 or 28) forms a connector attachment (6) to the free end of which a Y-piece (3) having an inhalation hose nozzle (4) and an exhalation hose nozzle (5), the second connecting element (2) forming a connector (7) with a tube attachment for an endotracheal tube.

19. An apparatus according to Claim 18, characterised in that the connector attachment (6) for the Y-piece (3) has at one end portion a screw thread (27) for establishing a screwed connection to the Y-piece (3).

20. An apparatus according to one of Claims 18 or 19, characterised in that the inhalation hose nozzle (4) and the exhalation hose nozzle (5) of the Y-piece (3) form respectively third and fourth plug-in connection elements with at their ends plug-in connection portions which are constructed in keeping with the plug-in connection portion (9) of the connector (7) and in that fifth and sixth plug-in connection elements (28, 29) are provided in the form of hose attachments, of which the respective end portions of the plug-in connection are constructed in accordance with the plug-in connection portion of the connector attachment (6).

21. An apparatus according to one of Claims 18 to 20, characterised in that the first, fifth and sixth plug-in connection elements (6, 28, 29) have in each case an O-ring seal (30) in their inner wall zones which follow on from the securing and releasing elements (11 , 12).

**Revendications**

1. Appareil respiratoire ou d'anesthésie, avec des conduits de gaz et un système de connexion pour ces conduits de gaz, ce système de connexion présentant au moins un assemblage par emboîtement qui est constitué de deux éléments d'assemblage sensiblement tubulaires (1,2) qui peuvent être emboîtés l'un dans l'autre, un premier (1) des éléments d'assemblage présentant un élément de blocage (11) qui s'engage sous action élastique dans une rainure annulaire (10) qui fait le tour de la paroi extérieure d'un second (2) des éléments d'assemblage, et présentant un élément de déverrouillage (12) à actionnement manuel, qui sert à déplacer l'élément de blocage (11) hors de la rainure annulaire entourante (10), et les éléments d'assemblage étant réalisés étanchéifiés l'un par rapport à l'autre et à rotation relative libre, caractérisé en ce que l'élément de blocage est réalisé sous la forme d'un secteur de jonc élastique (11) à élasticité propre, auquel est associé un premier évidement (13) de la paroi intérieure du premier (1 ou 28) des éléments d'assemblage, l'élément de déverrouillage est réalisé sous la forme d'un élément de déverrouillage (12) sensiblement plat qui est fixé sur le secteur de jonc élastique (11) et auquel est associé un deuxième évidement (18) de la paroi extérieure du premier (1 ou 28) des éléments d'assemblage, et l'élément de déverrouillage (12) est muni d'un cannelage (23) sur sa surface tournée vers l'extérieur et peut être déplacé en coulissement, dans la région du deuxième évidement (18), dans le sens périphérique du premier (1 ou 28) des éléments d'assemblage de telle sorte que le secteur de jonc élastique (11) peut être alternativement rentré dans le premier évidement (13), en vue du déverrouillage de l'assemblage, et ressorti de cet évidement, en vue du verrouillage de l'assemblage.

2. Appareil selon la revendication 1, caractérisé en ce que le premier évidement (13) de la paroi intérieure (15) du premier élément d'assemblage (1 ou 28) est réalisé sous la forme d'un secteur de rainure annulaire correspondant au secteur de jonc élastique (11).

3. Appareil selon la revendication 2, caractérisé en ce que le secteur de jonc élastique (11) est assemblé rigidement à la paroi intérieure (15) du premier élément d'assemblage (1 ou 28) à une (14) de ses extrémités, et est fixé à l'élément de déverrouillage (12) à son autre extrémité (16).

4. Appareil selon l'une des revendications 1 à 3, caractérisé en ce que le secteur de jonc élastique (11) présente une précontrainte qui définit la position de verrouillage.

5. Appareil selon l'une des revendications précédentes, caractérisé en ce que l'élément de déverrouillage (12) peut, à l'intérieur de l'évidement (18) de la paroi extérieure (17) du premier élément d'assemblage (1 ou 28) qui lui est associé, être déplacé sur une distance prédéterminée dans son sens circonférentiel entre deux butées finales (19,20), la première butée finale (19) correspondant à la position de verrouillage et la deuxième butée finale (20) correspondant à la position de déverrouillage.

6. Appareil selon la revendication 5, caractérisé en ce que le premier évidement (13), réalisé sous la forme d'un secteur de rainure annulaire dans la paroi intérieure du premier élément d'assemblage (1 ou 28), est relié, par l'intermédiaire d'une ouverture (21) pratiquée dans la paroi du premier élément d'assemblage (1 ou 28), au deuxième évidement (18) qui reçoit l'élément de verrouillage (12) et qui est pratiqué dans la paroi extérieure (17) du premier élément d'assemblage (1 ou 28).

7. Appareil selon la revendication 6, caractérisé en ce que des moyens de fixation (22) sont prévus qui, en traversant l'ouverture (21) de la paroi du premier élément d'assemblage (1 ou 28), fixent l'élément de déverrouillage (12) à l'extrémité correspondante (16) du secteur de jonc élastique (11).

8. Appareil selon la revendication 7, caractérisé en ce que les moyens de fixation (22) pour l'élément de déverrouillage (12) sont montés de manière amovible.

9. Appareil selon la revendication 7 ou 8, caractérisé en ce que l'élément de déverrouillage (12) est réalisé sous la forme d'une rondelle coulissante.

10. Appareil selon la revendication 8 ou 9, caractérisé en ce que l'autre extrémité (16) du secteur de jonc élastique (11), qui est prévue pour la fixation avec l'élément de déverrouillage (12), présente un alésage fileté (24), et l'élément de déverrouillage (12) luimême présente une ouverture (25) disposée de manière correspondante, et en ce que les moyens de fixation (22) sont constitués par une vis qui permet de fixer l'élément de déverrouillage (12) au secteur de jonc élastique (11) dans la région de l'ouverture (21) de la paroi du premier élément d'assemblage (1 ou 28).

11. Appareil selon l'une des revendications 2 à 10, caractérisé en ce que le premier évidement (13), réalisé en forme de secteur de rainure annulaire dans la paroi intérieure (15) du premier élément d'assemblage (1), est délimité vers l'extérieur, dans sa partie d'assemblage par emboîtement qui est associée à l'extrémité libre de l'élément d'assemblage, par un anneau de fermeture (26) qui peut être inséré dans un évidement correspondant, prévu du côté terminal de la paroi intérieure du premier élément d'assemblage (1).

12. Appareil selon la revendication 11, caractérisé en ce qu'une broche de fixation sert à fixer l'une des extrémités(14) du secteur de jonc élastique (11) à la paroi intérieure (15) du premier élément d'assemblage (1), broche pour laquelle des perçages d'introduction d'axes parallèles sont prévus tant dans l'extrémité concernée du secteur de jonc élastique que dans la partie de paroi du premier élément d'assemblage qui se trouve dans la région de l'extrémité correspondante du secteur de rainure annulaire.

13. Appareil selon la revendication 12, caractérisé en ce que la broche de fixation sert également à fixer l'anneau de fermeture (26), qui présente à cet effet lui-aussi un perçage d'introduction, d'axe parallèle, pour la broche de fixation.

14. Appareil selon la revendication 12 ou 13, caractérisé en ce que la broche de fixation est une broche métallique, notamment une broche en acier.

15. Appareil selon l'une des revendications précédentes, caractérisé en ce que le secteur de jonc élastique (11) est réalisé en métal enrobé de matière plastique, notamment en acier enrobé de matière plastique.

16. Appareil selon l'une des revendications 1 à 14, caractérisé en ce que le secteur de jonc élastique (11) est réalisé exclusivement en matière plastique.

17. Appareil selon l'une des revendications précédentes, caractérisé en ce que le secteur de jonc élastique (11) s'étend sur un arc de cercle de radian 3/2 $\pi$.

18. Appareil selon l'une des revendications précédentes, caractérisé en ce que le premier élément d'assemblage (1 ou 28) constitue un embout de connecteur (6) à l'extrémité libre duquel est raccordé un raccord en Y (3) muni d'une olive d'inspiration (4) et d'une olive d'expiration (5), et le deuxième élément d'assemblage (2) constitue un connecteur (7) à embout tubulaire, pour un tube endo-trachéal.

19. Appareil selon la revendication 18, caractérisé en ce que l'embout de connecteur (6) pour le raccord en Y (3) présente, sur une de ses parties terminales, un filetage (27) pour réaliser un assemblage vissé avec le raccord en Y (3).

20. Appareil selon la revendication 18 ou 19, caractérisé en ce que l'olive d'inspiration (4) et l'olive d'expiration (5) du raccord en Y (3) constituent respectivement un troisième et un quatrième éléments d'assemblage par emboîtement, avec des parties terminales d'assemblage par emboîtement qui sont réalisées conformément à la partie d'assemblage par emboîtement (9) du connecteur (7), et en ce qu'un cinquième et sixième éléments d'assemblage par emboîtement (28,29) sont prévus sous la forme d'embouts d'olives, dont les parties terminales respectives d'assemblage par emboîtement sont réalisées conformément à la partie d'assemblage par emboîtement de l'embout de connecteur (6).

21. Appareil selon l'une des revendications 18 à 20, caractérisé en ce que les premier, cinquième et sixième éléments d'assemblage par emboîtement (6,28,29) présentent, dans une région de paroi intérieure qui fait suite aux éléments de blocage et de déverrouillage (11,12), un joint d'étanchéité annulaire respectif (30).

4

5

3

**Fig. 1**

31

30

6 27 1 16 11

18 15 14

26 17 13

26

**Fig. 1b** 11 26

**Fig. 1a**

10 9

7 2

8

27

6

1 17

1 21

**Fig. 2**

Fig. 5

Fig. 6

Fig. 3

Fig. 4

Fig. 7

Fig.8